# EUROPEAN PATENT SPECIFICATION

(11) **EP 1 612 275 B1**
(45) Date of publication and mention of the grant of the patent: **26.11.2008**
(21) Application number: 05254051.5
(22) Date of filing: 29.06.2005
(51) Int. Cl.: C12Q 1/00, G01N 33/558, G01N 33/543

(54) **Methods and compositions for characterizing a redox reagent system enzyme**
Verfahren und Zusammensetzungen zur Charakterisierung eines Redox-Reagenz-System Enzyms
Méthodes et compositions pour caractériser une enzyme d' un système réactif rédox

(30) Priority: 30.06.2004 US 883629
(43) Date of publication of application: 04.01.2006
(73) Proprietor: LifeScan, Inc., Milpitas, CA 95035-6312 (US)
(72) Inventor: Byrd, Patricia, Gilroy, California 95020 (US); Qian, Suyue, Fremont, California 94539 (US); Hartz, Thomas P., Los Gatos, California 95032 (US)
(74) Representative: Mercer, Christopher Paul

(56) References cited:
- EP-A- 1 324 038
- EP-A- 1 398 386
- WO-A-03/012422
- US-A- 5 055 195
- CHAUBEY A ET AL: "Mediated biosensors" BIOSENSORS & BIOELECTRONICS, ELSEVIER SCIENCE PUBLISHERS, BARKING, GB, vol. 17, no. 6-7, June 2002 (2002-06), pages 441-456, XP002251006 ISSN: 0956-5663

## Description

### INTRODUCTION

### Background

Analyte detection in physiological fluids or samples, e.g., blood or blood-derived products, is of ever increasing importance to today's society. Analyte detection assays find use in a variety of applications, including clinical laboratory testing, home testing, etc., where the results of such testing play a prominent role in the diagnosis and management of a variety of disease conditions. Analytes of interest include glucose for diabetes management, cholesterol, and the like. In response to this growing importance of analyte detection, a variety of analyte detection protocols and devices for both clinical and home use have been developed.

One type of method that is employed for analyte detection is an electrochemical method. In such methods, an aqueous liquid sample is placed into a reaction zone in an electrochemical cell comprising at least two electrodes, i.e., a reference and working electrode. The component to be analyzed is allowed to react directly with an electrode, or directly or indirectly with a redox reagent to form an oxidizable (or reducible) substance in an amount corresponding to the concentration of the component to be analyzed, i.e., analyte. The quantity of the oxidizable (or reducible) substance present is then estimated electrochemically and related to the amount of analyte present in the initial sample.

In many such electrochemical approaches to analyte detection, an analyte oxidizing signal producing system comprising an enzyme component and a mediator component is employed, where the enzyme component oxidizes the analyte of interest and then transfers an electron to a mediator which, in turn, transfers the electron to an electrode in the electrochemical cell, thereby generating an electrical signal from which the analyte concentration can be determined.

In designing electrochemical analyte detection assays and systems, it is desirable to employ an analyte specific enzyme. For example, for glucose determination, the soluble form of pyrroloquinoline quinone (PQQ)-dependent glucose dehydrogenase (hereinafter referred to s-GDH) is one enzyme that can be employed in a glucose oxidizing signal producing system. s-GDH is a poor electron acceptor and is therefore beneficially insensitive to the presence of oxygen in fluid samples.

However, a disadvantage of naturally occurring or "wild-type" s-GDH is that it oxidizes not only glucose but also other reducing sugars including maltose, galactose, lactose, mannose, xylose and ribose. The reactivity of s-GDH towards sugars other than glucose may, in certain cases, impair the accuracy of determining blood glucose levels in some diabetic patients. For example, patients on peritoneal dialysis treated with icodextrin (a glucose polymer) may contain high levels of maltose in their blood.

This lack of specificity of s-GDH has lead to an effort to improve the specificity of s-GDH by generating "mutant" forms of the enzyme, e.g., where one or more amino acid substitutions have been made. However, before such "altered" enzymes can be employed, they must be tested to determine their suitability for use in analyte detection assays.

While time-consuming, spectrophotometric assays have been used to determine the activity of s-GDH, but are used only in enzyme-limiting conditions. A spectrophotometric assay using 2,6-dichlorophenolindophenol (DCIP) and phenazine methosulfate (PMS) as artificial mediators is described in U.S. Patent 6,103,509 and European Patent Application No. EP 1 176 202 A1.

However, typical commercial glucose test strips using s-GDH do not use enzyme-limiting conditions because enzyme is present in excess to ensure long-term strip performance. Recombinant proteins, for example, mutant forms of s-GDH, are generally produced in only small quantities. Testing the reactivity of these mutant forms of s-GDH involves making enzyme-coated strips and testing each lot of strips with whole blood or plasma spiked with glucose and each possible interfering (i.e., reducing) sugar at multiple concentrations. Production of test strips coated with each mutant form of s-GDH requires large amounts of each mutant, is time and labor intensive and requires that technicians test the strips with human blood or plasma, possible biohazards.

Accordingly, a rapid and inexpensive assay that is substrate limiting is needed to determine if mass production of a particular mutant enzyme, e.g., s-GDH, is warranted due to its improved glucose specificity over wild-type s-GDH. The assay should also be applicable to non-blood containing samples spiked with glucose or interfering sugars rather than whole blood or plasma samples spiked with reducing sugars. Still needed in the field, therefore, is a method that is rapid and simple to use for determining the carbohydrate specificity of mutant forms of s-GDH that uses small amounts of enzyme and that models substrate-limiting conditions on a typical electrochemical-based test strip for measuring an analyte (e.g., glucose) in a fluid sample (e.g., whole blood or plasma). In addition, a rapid and simple method for measuring enzyme activity is also desired.

### Relevant Literature

U.S. Patent documents: 5,484,708; 5,723,284; 5,834,224; 5,942,102; 5,912,199; 5,997,817; 6,059,946; 6,083,710; 6,103,509; 6,121,009; 6,134,461; 6,179,979; 6,193,973; 6,231,531; 6,284,125; 6,340,428; 6,444,115 and 6,716,577; as well as other patent documents: US 2003/0104595; WO 99/49307; WO 97/18465; WO 01/57510; WO 01/57238; WO 02/48707; WO 02/50609; WO 02/06788; EP0969097; EP1176202; JP091403378A; and GB 2 304 628.

### SUMMARY OF THE INVENTION

Methods and compositions for characterizing an enzyme of a redox reagent system are provided. In practicing the subject methods, a sample that includes an enzyne of a redox reagent system and a known amount of substrate is applied to an electrochemical cell that includes an enzyme-free reagent composition having a redox reagent system mediator. Also provided are electrochemical test strips that include the subject electrochemical cells, and systems and kits that include the same. The subject invention finds use in a variety of different applications, including redox reagent system characterization applications.

### BRIEF DESCRIPTION OF THE FIGURES

A better understanding of the features and advantages of the present invention will be obtained by reference to the following detailed description that sets forth illustrative embodiments, in which the principles of the invention are utilized, and the accompanying drawings, of which:
FIG. 1 is a flowchart illustrating a sequence of steps in a process according to an exemplary embodiment of the present invention for testing mutant forms of s-GDH;
FIGS. 2A and 2B are exploded and top views, respectively, of a test strip as may be used in exemplary processes according to the present invention;
FIG. 3 is a flowchart illustrating a sequence of steps in a process according to an exemplary embodiment of the present invention for measuring the activity of wild-type s-GDH and mutants thereof;
FIG. 4 is a graph showing the absolute bias of wild-type s-GDH and two mutant forms of s-GDH (Asn452Thr and Asp167Glu) to a control containing no interfering sugars as a function of xylose concentration using a conventional enzyme-coated test strip;
FIG. 5 is a graph showing response as a function of sugar concentration for a conventional enzyme-coated test strip in which the enzyme is wild type s-GDH;
FIG. 6 is a graph showing response as a function of sugar concentration using wild-type s-GDH in a process according to the present invention;
FIG. 7 is a graph showing response as a function of sugar concentration using a mutant form of s-GDH, Asn452Thr, in a process according to the present invention;
FIG. 8 is a graph showing response as a function of sugar concentration using another mutant form of s-GDH, Asp167Glu, in a process according to the present invention;
FIG. 9 is a graph showing a standard curve for wild-type s-GDH obtained in a process according to the present invention;
FIG. 10 is a graph showing the standard curve illustrated in FIG. 9 as a double reciprocal plot.

### DESCRIPTION OF THE SPECIFIC EMBODIMENTS

Methods and compositions for characterizingan enzyme of a redox reagent system are provided. In practicing the subject methods, a sample that includes an enzyme of a redox reagent system and a known amount of substrate is applied to an electrochemical cell that includes an enzyme-free reagent composition having a redox reagent system mediator. Also provided are electrochemical test strips that include the subject electrochemical cells, and systems and kits that include the same. The subject invention finds use in a variety of different applications, including redox reagent system characterization applications.

The scope of the present invention will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and a re also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

All publications mentioned herein are incorporated herein by reference for the purpose of describing and disclosing the cell lines, vectors, and methodologies, which are described in the publications, which might be used in connection with the presently described invention.

As summarized above, the subject invention provides methods and compositions for characterizing an enzyme. In further describing the subject invention, the subject methods and representative specific applications thereof are reviewed first in greater detail, followed by a review of representative compositions and systems that find use in practicing the subject methods.

### METHODS

As summarized above, the subject invention provides methods of characterizing an enzyme. By characterizing an enzyme is meant determining a feature or parameter of the enzyme. The feature may be a feature that is inherent to the enzyme, e.g., its substrate selectivity, or a feature that, at least in part, dependent on its environment, such as the concentration of the enzyme in a given fluid medium, the activity of an enzyme in a given fluid medium etc. Of course, the feature may be a product of both inherent and environmental factors. Specific representative characteristics that may be determined using the methods of the subject invention are described in greater detail below.

The methods include applying a redox reagent system enzyme containing sample to an electrochemical cell and detecting an electrical signal generated by the cell, where the detected signal is then employed to characterize the redox reagent system enzyme in the sample.

A feature of certain representative embodiments of the invention is that the sample that is contacted with the electrochemical cell includes an enzyme of a redox reagent system and a known amount of an enzyme substrate, while the electrochemical cell to which the sample is applied includes an enzyme-free reagent composition that includes a mediator component of the redox reagent system of which the enzyme in the sample is a member. The sample and electrochemical cell components employed in these embodiments of the subject methods will now be described separately in greater detail.

### Sample

The sample that is contacted with the electrochemical cell in practicing the subject methods is one that includes an enzyme of a redox reagent system and a known amount of a substrate or at least potential substrate for the enzyme, i.e., an enzyme substrate.

### Enzyme

The enzyme component of the sample, in many embodiments, an enzyme or plurality of enzymes that work in concert to oxidize an analyte of interest. In other words, the enzyme member may be made up of a single analyte oxidizing enzyme or a collection of two or more enzymes that work in concert to oxidize a given analyte of interest, allowing generation of a electrochemical signal in an electrochemical cell, as described below. Enzymes of interest include oxidases, dehydrogenases, lipases, kinases, diaphorases, quinoproteins and the like. More specific representative enzymes of interest include, but are not limited to: glucose oxidase, glucose dehydrogenase, cholesterol esterase, cholesterol oxidase, lipoprotein lipase, glycerol kinase, glycerol-3-phosphate oxidase, lactate oxidase, lactate dehydrogenase, pyruvate oxidase, alcohol oxidase, bilirubin oxidase, uricase, and the like. In certain embodiments of interest, the enzyme is a glucose dehydrogenase, such as a s-GDH or a mutant thereof, as described in greater detail below.

In certain embodiments, the enzyme component may be present in a concentration ranging from about 35 to about 450, usually from about 80 to about 270 µM.

### Enzyme Substrate

The sample employed in the subject methods also includes a known amount of an enzyme substrate. The enzyme substrate may be a known substrate for the enzyme present in the sample or a candidate substrate for the enzyme present in the sample. For example, where the enzyme is a glucose dehydrogenase, the substrate in the sample may be glucose (which is known to be a substrate for the enzyme) or another sugar, e.g., maltose, that is either known to also be a substrate for the enzyme or suspected to be a substrate for the enzyme. By known amount is meant that the sample has a defined or predetermined quantity or concentration of substrate. In certain embodiments, the concentration of enzyme substrate in the sample ranges from about 0 to about 50 mM (about 0 to about 900 mg/dL), usually from about 0 to about 45 mM (about 0 to about 810 mg/dL).. In certain embodiments when the enzyme substrate is definitely present, the concentration of the enzyme substrate in the sample ranges from about 0.1 to about 50 mM (e.g., from about 0.01 to about 900 mg/dL), usually from about 0.1 to about 45 mM (e.g., from about 0.01 to about 810 mg/dL).

### Coenzyme

In certain embodiments, the sample of fluid medium also includes a coenzyme, which activates the enzyme component. An example of a coenzyme of interest is pyrroloquinoline quinone (PQQ). Other cofactors of interest include, but are not limited to: nicotinamide adenine dinucleotide (NAD), flavin adenine dinucleotide (FAD), cytochrome, and the like, depending on the type of enzyme applied to the test reagent. IN certain embodiments, the concentration of any coenzymes may range from about 60 to about 670 µM, usually from about 100 to about 430 µM.

### Enzyme Cofactor

In certain embodiments, the samples further include one or more enzyme cofactors. Enzyme cofactors of interest include divalent metal cations, e.g., Ca²⁺, Mg²⁺, etc. The concentration of any cofactors may range from about 0.5 to about 5 mM.

### Electrochemical Cell

As summarized above, in practicing the subject methods the sample is applied to an electrochemical cell that includes an enzyme-free reagent composition. A variety of different types of electrochemical cell configurations are known, including those described in U.S. Patent documents: 5,723,284; 5,834,224; 5,942,102; 5,972,199; 5,997,817; 6,083,710; 6,121,009; 6,134,461; 6,193,873; and 6,716,577; ; as well as other patent documents: WO 99/49307; WO 97/18465; WO 01/57510; WO 01/57238; WO 02/48707; WO 02/50609; EP 0 969 097A2 and GB 2 304 628; . Any of these or other electrochemical cells known to those of skill in the art may be modified to incorporate the subject compositions.

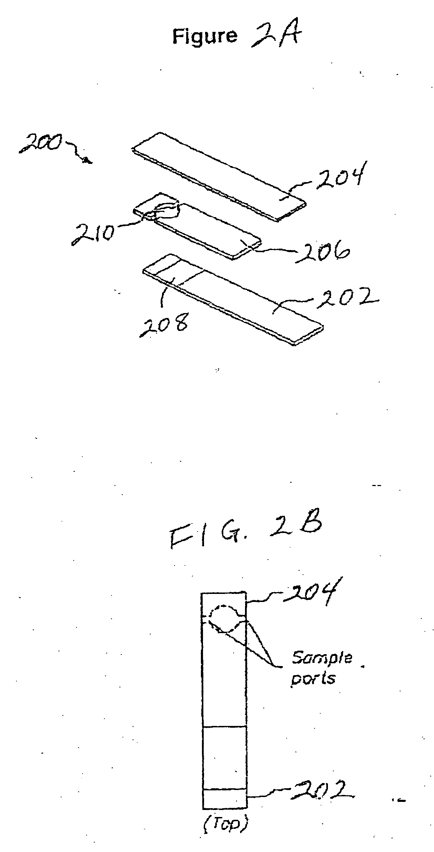

The electrochemical cell of the present invention is present in an electrochemical test strip. A representation of the electrochemical test strip according to the subject invention is provided in Figures 2A and 2B. Figure 2A provides an exploded view of the electrochemical test strip which is made up of working electrode and reference electrode separated by spacer layer which has a cutaway section that defines the reaction zone or area in the assembled strip, where these elements are further described below. Figure 2B shows the same test strip in assembled form. Each of the various components are now described in greater detail below.

### Electrodes

The subject electrochemical test strips comprising the reagent compositions include a working electrode and a reference electrode. Generally, the working and reference electrodes are configured in the form of elongated rectangular strips. Typically, the length of the electrodes ranges from about 1.9 to about 4.5 cm, usually from about 2 to about 2.8 cm. The width of the electrodes ranges from about 0.38 to about 0.76 cm, usually from about 0.51 to about 0.67 cm. The reference electrodes typically have a thickness ranging from about 10 to 100 nm and usually from about 18 to about 22 nm. In certain embodiments, the length of one of the electrodes is shorter than the length of the other electrode, wherein in certain embodiments it is about 0.32 cm shorter.

The working and reference electrodes are further characterized in that at least the surface of the electrodes that faces the reaction area in the strip is a conductive material, e.g., a metal or other conductive material, where representative materials of interest include, but are not limited to: palladium, gold, platinum, silver, iridium, carbon, doped tin oxide, stainless steel and the like. In certain embodiments, the conductive material is gold or palladium. While in principle the entire electrode may be made of the conductive material, each of the electrodes is generally made up of an inert support material on the surface of which is present a thin layer of the conducting material component of the electrode. Any convenient inert backing material may be employed in the subject electrodes, where typically the material is a rigid material that is capable of providing structural support to the electrode and, in turn, the electrochemical test strip as a whole. Suitable materials that may be employed as the backing substrate include plastics, e.g. PET, PETG, polyimide, polycarbonate, polystyrene, silicon, ceramic, glass, and the like.

### Spacer Layer

A feature of the subject electrochemical test strips is that the working and reference electrodes as described above face each other and are separated by only a short distance, such that the distance between the working and reference electrode in the reaction zone or area of the electrochemical test strip is extremely small. This minimal spacing of the working and reference electrodes in the subject test strips is a result of the presence of a thin spacer layer positioned or sandwiched between the working and reference electrodes. The thickness of this spacer layer generally ranges from about 1 to about 500 µm, usually from about 100 to about 200 µm. The spacer layer is cut so as to provide a reaction zone or area with at least an inlet port into the reaction zone, and generally an outlet port out of the reaction zone as well. A representative spacer layer configuration can be seen in Figs. 2A and 2B. While the spacer layer is shown in these figures as having a circular reaction area cut with side inlet and outlet vents or ports, other configurations are possible, e.g., square, oval, triangular, rectangular, irregular shaped reaction areas, etc. The spacer layer may be fabricated from any convenient material, where representative suitable materials include PET, PETG, polyimide, polycarbonate and the like, where the surfaces of the spacer layer may be treated so as to be adhesive with respect to their respective electrodes and thereby maintain the structure of the electrochemical test strip. Of particular interest is the use of a die-cut double-sided adhesive strip as the spacer layer.

### Reaction Zone

The subject electrochemical test strips include a reaction zone or area that is defined by the working electrode, the reference electrode and the spacer layer, where these elements are described above. Specifically, the working and reference electrodes define the top and bottom of the reaction area, while the spacer layer defines the walls of the reaction area. The volume of the reaction area is at least about 0.1 µl, usually at least about 1 µl and more usually at least about 1.5 µl, where the volume may be as large as 10 µl or larger. As mentioned above, the reaction area generally includes at least an inlet port, and in many embodiments also includes an outlet port. The cross-sectional area of the inlet and outlet ports may vary as long as it is sufficiently large to provide an effective entrance or exit of fluid from the reaction area, but generally ranges from about 9×10⁻⁵ to about 5×10⁻³ cm², usually from about 5×10⁻⁴ to about 2.5×10⁻³ cm².

### Enzyme-Free Reagent Composition

Present in the reaction zone is an enzyme-free reagent formulation, where the reagent formulation is typically present in a dry format. By enzyme-free is meant that the formulation does not include at least the redox reagent system enzyme that is present in the sample to be assayed by the electrochemical cell. As such, if the sample to be applied to the electrochemical cell includes a glucose dehydrogenase, the reagent formulation present in the electrochemical cell does not include a glucose dehydrogenase, and in many embodiments does not include any enzymes.

A feature of the enzyme-free reagent formulations present in the electrochemical cells is the presence of a redox mediator, which may comprise one or more mediator agents. The mediator acts an intermediary that facilitates the transfer of electrons from the enzyme (which has taken one or more electrons from the analyte during analyte oxidation) to the electrode. A variety of different mediator agents known in the art may be used, including ferricyanide, phenazine ethosulphate, phenazine methosulfate, phenylenediamine, N,N,N',N'-tetramethyl phenylenediamine, 1-methoxy-phenazine methosulfate, 2,5-dimethyl-1,4-benzoquinone, 2,6-dimethyl-1,4-benzoquinone, 2,5-dichloro-1,4-benzoquinone, ferrocene derivatives, osmium bipyridyl complexes, ruthenium complexes and the like. In representative embodiments, the redox mediator is ferricyanide.

Another component of the reagent composition is a mediator stabilizing buffering component. The subject mediator stabilizing buffering components may be made up of one or more, e.g., two, three, four or more, distinct buffering agents, where the buffering component stabilizes the mediator during storage of the composition in dry form such that little if any of the mediator is reduced prior to use, e.g., during storage.

In one embodiment, the buffer agents are polycarboxylic acids. By polycarboxylic acids is meant that the buffering agents include two or more carboxylic acid functional moieties, where the number of different carboxylic acid functional moieties may range from about 2 to about 10, e.g., from about 2 to about 8, including from about 2 to about 6. The carboxylic acid groups or functional moieties of the subject buffering agents may be attached to a number of different structures, including aliphatic, alicyclic, aromatic and heterocyclic structures. The presence of more than one carboxylic acid group can have the beneficial effect of providing at least one pKa value for the buffer in the desired range. Specific polycarboxylic acids of interest include, but are not limited to: mellitic acid, citraconic acid, maleic acid, and the like, etc.

Where the reagent composition is a dry reagent formulation, e.g., as may be present in an electrochemical test strip as described in greater detail below, the amount of buffering component present in the dry composition typically ranges from about 0.01 to about 40.00, usually from about 1 to about 10% wt/wt.

The reagent composition may further include one or more of the following additional components: a wetting agent, detergent stabilizer, viscosity modifier or combinations thereof.

A wetting agent may be added, in some embodiments in combination with a detergent, to the reagent composition to facilitate uniform coating of the reagent composition onto an electrochemical test strip. A plurality of one or more of the combination of agents may also be used. The agents used may improve dissolution of the assay reagents as well as enhance the wicking properties of a capillary fill strip. The agents include those known in the art, for example, polymers, anti-foaming agents, and surfactants. Representative types of surfactants/detergents of interest include, but are not limited to: Tritons, Macols, Tetronics, Silwets, Zonyls, and Pluronics. Suitable agents include Pluronic materials which are block co-polymers of polyethylene oxide and polypropylene oxide. Examples of Pluronic materials indude Pluronic P103 which has good wetting properties and Pluronic F87 Prill which has good detergent properties. Both Pluronic P103 and F87 Prill also have a cloud point temperature greater than 80 °C which is desirable since this property avoids a phase change in the composition during the drying process.

Stabilizers may also be added to the reagent composition to help stabilize the enzyme and prevent denaturation of the protein. The stabilizer may also help stabilize the redox state of the mediator, in particular, the oxidized redox mediator. Examples of stabilizing agents include, but are not limited to: carbohydrates (e.g., sucrose, trehalose, mannitol, and lactose), amino acids, proteins (such as BSA and albumin) and organic compounds such as EDTA and the like.

Viscosity modifiers may also be added to the reagent to modify the liquid reagent rheology. Examples of such agents include poly(acrylic acid), poly(vinyl alcohol), dextran, BSA and the like.

### Methods

In practicing the subject methods, the first step is to introduce a quantity of the fluid medium or sample into the reaction area of an electrochemical cell, e.g., of an electrochemical test strip. The time period between sample preparation and application to the reaction area may vary, but in certain representative embodiments ranges from about 30 seconds to about 8 hours, such as from about 5 minutes to about 3 hours, including from about 30 minutes to about 60 minutes. The amount of sample that is introduced into the reaction area of the electrochemical cell may vary, but generally ranges from about 0.05 to about 10 µl, usually from about 0.5 to about 1.6 µl. The sample may be introduced into the reaction area using any convenient protocol, where the sample may be injected into the reaction area, allowed to wick into the reaction area, and the like, as may be convenient.

Following application of the sample to the reaction zone, an electrochemical measurement is made using the reference and working electrodes. The electrochemical measurement that is made may vary depending on the particular nature of the assay and the device with which the electrochemical test strip is employed, e.g. depending on whether the assay is coulometric, amperometric or potentiometric. Generally, the electrochemical measure will measure charge (coulometric), current (amperometric) or potential (potentiometric), usually over a given period of time following sample introduction into the reaction area. Methods for making the above described electrochemical measurement are further described in U.S. Patent Nos.: 4,224,125; 4,545,382; and 5,266,179; as well as WO 97/18465; WO 99/49307.

Following detection of the electrochemical signal generated in the reaction zone as described above, the signal is employed to characterize the enzyme of the sample in some manner, e.g., to determine the substrate specificity of the enzyme, to determine the concentration of the enzyme in the sample applied to the cell, etc., where these two representative applications are described in greater detail below. In certain embodiments, the electrochemical signal measurement steps and enzyme characterization steps, as described above, are performed automatically by a device designed to work with the test strip to perform such steps following application of a sample to the electrochemical cell. A representative reading device for automatically practicing at least certain of these steps, is further described in U.S. Patent No. 6,193,873.

### UITILITY

As indicated above, the subject methods find use in a variety of different applications, where representative applications in which the subject methods find use are enzyme characterization applications, as described above.

A first representative enzyme characterization application that may be accomplished by the subject methods is determining the substrate or analyte specificity of a first enzyme as compared to one or more additional enzymes, e.g., the specificity of a mutant glucose dehydrogenase for glucose as compared to other reducing sugars, e.g., maltose, where the specificity is determined relative to a second glucose dehydrogenase, e.g., wild-type dehydrogenase.

In practicing these representative applications of the subject invention, a set of fluid samples that at least includes first and second enzymes combined with first and second enzyme substrates is prepared and each prepared sample is then individually tested in an electrochemical cell. By individually tested is meant that the each sample of the set is tested in its own or separate electrochemical cell.

In certain of these embodiments, the set of samples that is prepared and then individually applied to an electrochemical cell according to the present methods at least includes:
(i) a sample containing a first redox reagent system enzyme and a known first concentration of a first enzyme substrate;
(ii) a sample containing the first redox reagent system enzyme and a known first concentration of a second enzyme substrate;
(iii) a sample containing a second redox reagent system enzyme and said known first concentration of the first enzyme substrate; and
(iv) a sample containing the second redox reagent system enzyme and said known first concentration of the second enzyme substrate.

In certain embodiments, the specificity assay includes testing each of the enzymes at different concentrations of the first and second enzyme substrates, e.g., at a plurality of different concentrations of the first and second enzymes. In such embodiments, at least the following additional samples are prepared and tested:
(i) a sample containing the first redox reagent system enzyme and a known second concentration of the first enzyme substrate;
(ii) a sample containing the first redox reagent system enzyme and a known second concentration of the second enzyme substrate;
(iii) a sample containing the second redox reagent system enzyme and the known second concentration of the first enzyme substrate; and
(iv) a sample containing the second redox reagent system enzyme and the known second concentration of the second enzyme substrate.

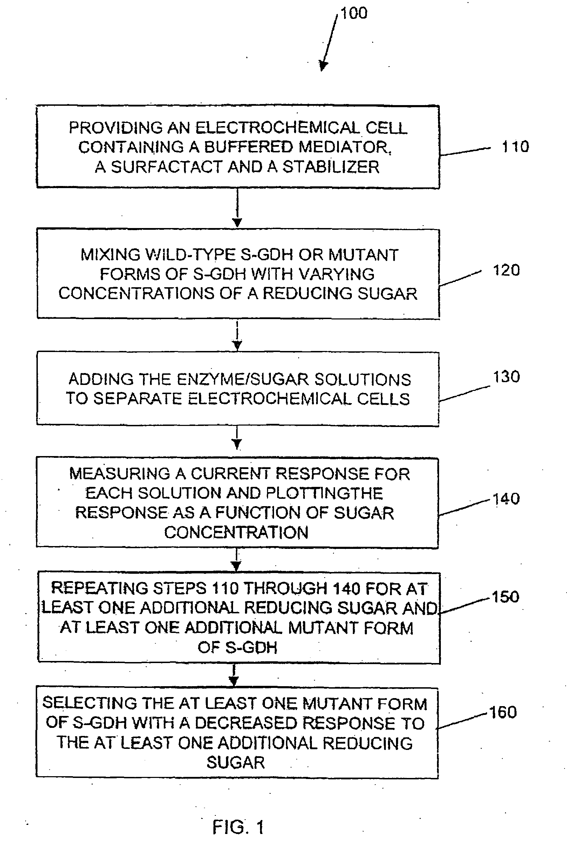

A representative embodiment of these applications is the evaluation of different s-GDH enzymes with respect to substrate specificity, i.e. ability to employ glucose but not competing reducing sugars as a substrate. This embodiment is further illustrated in the FIG 1. FIG. 1 is a flow chart of a process 100 for testing wild-type and mutant forms of s-GDH in accordance with an exemplary embodiment of the present invention. Process 100 includes providing an electrochemical cell, as set forth in step 110. A typical electrochemical cell as may be used with processes according to the present invention is provided in the form of a test strip 200, as shown in exploded and top views in FIGS. 2A and 2B, respectively. Test strip 200 includes a working electrode 202 and a reference electrode 204 separated by a spacer layer 206. Working electrode 202 includes a band of dried reagent 208 including a buffered mediator, a surfactant and a stabilizing agent. Spacer layer 206 includes a cutaway section that defines the reaction zone in electrochemical cell 210 in the assembled strip.

As reviewed above, suitable materials for reference electrode 204 include those listed above for working electrode 202. The material for reference electrode 204 in an opposing electrode format is usually palladium or gold. In an electrochemical cell format in which the electrodes are coplanar, reference electrode 204 is usually carbon.

The reference electrode 204 is usually coated with a stabilizer containing a sulfur moiety in its molecular structure. The coating can also include a hydrophilic group and a spacer between the sulfur containing moiety and the hydrophilic group. Examples of compounds useful in reference electrode 204 coatings include, but are not limited to, 2-mercaptoethane sulfonic acid, 2-mercaptoethanol, 2-mercaptoethylamine, 3-mercaptoproprionic acid, thiophene, 4-carboxythiphene, cysteine, homocysteine, and cystine. Reference electrode 204 is usually comprised of gold coated with 2-mercaptoethane sulfonic acid.

Dried reagent 208 can be coated onto working electrode 202 by slot coating as described in European Patent Application EP 1324038A2, the disclosure of which is herein incorporated by reference. Other methods of coating reagent include, but are not limited to, ink-jetting and needle coating.

Spacer layer 206 is cut so as to provide a reaction zone with at least an inlet port into the reaction zone and generally an outlet port out of the reaction zone. Spacer layer 206 is shown in FIGS. 2A and 2B as having a circular reaction area cut with side inlet and outlet vents. Other reaction area configurations include, but are not limited to, square, oval, triangular, rectangular, and irregular shaped reaction areas. Spacer layer 206 can be fabricated from any suitable material including, but not limited to, PET, PETG, polyimide, and polycarbonate, whereby the surfaces of spacer layer 206 can be treated so as to be adhesive. Spacer layer 206 is typically a die-cut double-sided adhesive.

The mediator can be ferricyanide; phenazine ethosulphate; phenazine methosulfate; phenylenediamine; N,N,N',N'-tetramethyl phenylenediamine; 1-methoxy-phenazine methosulfate; 2,5-dimethyl-1,4-benzoquinone; 2,6-dimethyl-1,4-benzoquinone;, 2,5-dichloro-1,4-benzoquinone; ferrocene derivatives; osmium bipyridyl complexes; ruthenium complexes; or the like. Suitable buffering agents have little, if any, binding affinity for divalent metal cations (e.g., Ca2+) and can be citraconate, citrate, malic, maleic, phosphate, "Good" buffers or the like. In addition, the buffered solution may contain surfactants or wetting agents including Triton, Macol, Tetronic, Silwet, Zonyl, or Pluronic; and stabilizing agents including sucrose, trehalose, or mannitol. In the preferred embodiment the mediator is ferricyanide, the buffer is citraconate, the surfactant is Pluronic, and the stabilizing agent is sucrose.

Next, as set forth in step 120 in process 100, wild-type or at least one mutant form of s-GDH in a buffered solution is mixed with a coenzyme, a cofactor and varying concentrations of a first reducing sugar (e.g., glucose). The first reducing sugar can be, but is not limited to, glucose, galactose, maltose, xylose, or lactose. The coenzyme can be PQQ and the cofactor can be calcium.

The s-GDH employed in the processes according to the present invention, including process 100 and 300 (see below), can be any native or mutant form of s-GDH in which at least one modification in the amino acid sequence of the native enzyme is made including, but not limited to, making one or more amino acid substitutions or deletions to improve the glucose specificity. Techniques known to those skilled in the art can be used to make these modifications to s-GDH and are discussed in the aforementioned U.S. Patent No. 6,103,509 and European Patent Application No. EP 1 176 202 A1, where general methods of making mutants of a wild type enzyme are well-known to those of skill in the art.

As set forth in step 130 of FIG. 1, the enzyme/sugar solutions are each added to separate electrochemical cells containing buffered mediator, surfactant and stabilizing agent. Next, the current response is measured and is graphed as a function of sugar concentration, as set forth in steps 140. Keeping the enzyme/sugar solution separate from the mediator prior to measuring the current response is beneficial because of the fast reaction rate between the enzyme and mediator. If the enzyme and mediator are mixed prior to adding the solution to the electrochemical cell, the reaction will be complete or near completion before the current response can be measured. Steps 110 through 140 are then repeated for at least one additional reducing sugar and at least one additional mutant forms of s-GDH, as set forth in step 150 (see Examples 2-4). The at least one additional reducing sugar can be, but is not limited to, galactose, maltose, xylose, or lactose. Next, at least one mutant form of s-GDH with a decreased response to the at least one additional sugar is selected, as set forth in steps 160 and 170.

In this manner, the specificity of the mutant s-GDH relative to wild-type s-GDH may be readily determined.

In another representative application, the subject methods are employed to determine the concentration or activity of an enzyme in a fluid sample. In practicing these methods, a fluid sample containing an unknown amount of enzyme and a known amount of substrate is applied to an electrochemical cell. Following application, the detected electrical signal is used to determine the concentration of analyte in the applied sample, e.g., by comparing said detected electrical signal to a reference.

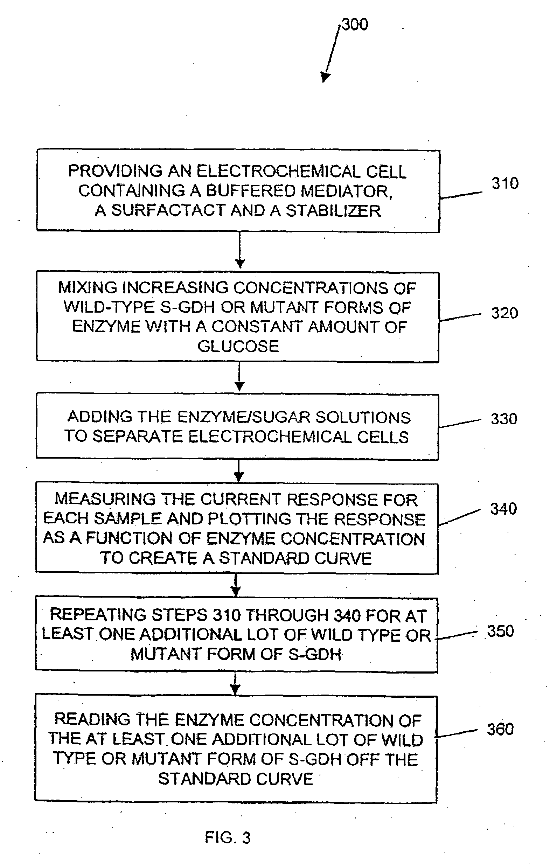

FIG. 3 is a flowchart illustrating a sequence of steps in a process 300 according to the present invention for measuring the activity of wild-type s-GDH and mutants thereof in a sample. Process 300 includes providing an electrochemical cell containing dried mediator in buffer with a surfactant and a stabilizer, as set forth in step 310. Next, increasing concentrations of wild-type s-GDH or a mutant form of s-GDH are mixed with a constant amount of glucose, as set forth by step 320. Each enzyme/glucose sample is then added to a separate electrochemical cell, as set forth in step 330. As set forth in step 340, the current response is measured for each sample and the response is graphed as a function of enzyme concentration to create a standard curve, which can be employed as a reference in the subsequent steps. Next, steps 310 through 340 are repeated for at least one additional lot of wild type or mutant form of s-GDH, as set forth in step 350. As set forth in step 360, the enzyme concentration of the at least one additional lot of wild type or mutant form of s-GDH is then read off the standard curve or reference, e.g., by comparing the detected signal to the reference.

### SYSTEMS

Also provided by the subject invention are systems for use in practicing the subject methods, where the systems include at least one sample or fluid medium that includes a redox reagent system enzyme and an electrochemical cell that includes an enzyme-free reagent composition, as described above.

The subject systems may also include a device for use in electrochemically assaying a sample using the subject reagent compositions.

The devices or meters of the subject systems are typically electrochemical measuring devices. The subject meters typically include: (a) a means for applying an electric potential to an electrochemical cell into which the sample has been introduced; and (b) a means for measuring cell current in the cell. Representative electrochemical meters or devices are described in U.S. Patent documents: 5,723,284; 5,834,224; 5,942,102; 5,972,199; 5,997,817; 6,083,710; 6,121,009; 6,134,461; and 6,193,873 as well as other patent documents: WO 99/49307; WO 97/18465; WO 01/57510; WO 01/57238; WO 02/48707; WO 02/50609; EP 0 969 097A2 and GB 2 304 628.

The following examples are offered by way of illustration and not by way of limitation.

### EXPERIMENTAL

### Example 1 ― Performance of conventional test strips coated with wild-type or mutant forms of s-GDH

In order to develop an assay for screening mutant forms of s-GDH with varying sugar specificities, the response of wild type and mutant forms of s-GDH with reducing sugars first were tested in a conventional enzyme-coated glucose test strip format. The screening assay ideally mimics the response shown with conventionally enzyme-coated strips tested with whole blood or plasma spiked with a reducing sugar. The mutant forms of s-GDH tested contained one amino acid substitution each: Asn452Thr and Asp167Glu. The test strips were made by a web-based process in which the working electrode (gold) was slot coated with a buffered reagent solution containing approximately 50 to 500 kU/ml wild type or mutant s-GDH, PQQ at a 2 to 1 mole ratio of PQQ to GDH, 2 mM CaCl₂, 750 mM ferricyanide, 67 mM citraconate at pH 6.8, 0.1% pluronics and 75 mM sucrose. The concentration of the wild type or mutant s-GDH in the reagent solution was adjusted such that approximately 13 activity units per electrochemical cell were used; hence, the activity units per ml of reagent solution varied depending on the activity units per gram of enzyme used. The activity of each enzyme was based on a spectrophotometric assay using DCIP (2,6-Dichlorophenolindophenol) and PES (Phenazine Ethosulfate) in 50mM PIPES (Piperazine, N, N' bis-(2-ethanesulfonic acid)) buffer at pH 6.8. In the assay, change in the absorbance of DCIP at 600nm was monitored and the decreasing rate of absorbance was referred to as the reaction rate of the enzyme. The enzyme activity by which 1 mmole of DCIP was reduced in one minute was defined as 1 Unit. The molar absorption coefficient of DCIP at pH 6.8 was 17.4 mM-1. A COBAS FARA II centrifugal analyzer was used for the spectrophotometric assay.

An IR energy source was used to dry the reagent on the working electrode, as is described in European Patent Application No. EP1324038, which is fully incorporated herein by reference. Electrochemical cells (or test strips) were formed by adhering the working electrode with dried reagent to a gold counter electrode containing dried MESA to prevent fouling of the counter electrode.

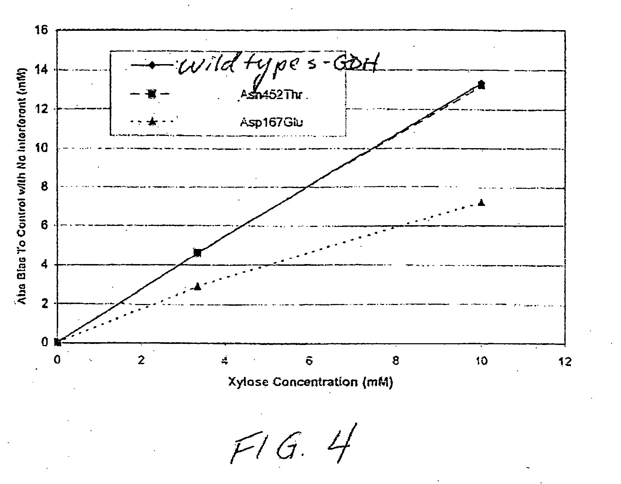

Whole blood at about 80 mg/dL (or 4.4 mM) of endogenous glucose was spiked with either a high therapeutic level (about 60mg/dL or 4 mM) or three times a high therapeutic level (about 180 mg/dL or 12 mM) of the interfering sugar xylose. The spiked samples were dosed onto the above test strips and glucose concentration was determined by chronoamperometry in which a potential of -0.3 V was applied for 10 seconds, followed by applying a potential of +0.3 V for 5 seconds. Absolute bias to a control sample with no xylose was calculated for each enzyme and was expressed as a function of xylose concentration, as shown in FIG. 4. There was a linear correlation between absolute bias and the interfering sugar xylose for wild type s-GDH and the Asn452Thr mutant, indicating that wild type s-GDH and Asn452Thr both recognize xylose as well as glucose. However, the Asp167Glu mutant exhibited a reduced response with xylose. Both mutant forms also demonstrated linear correlations between absolute bias and galactose or maltose (data not shown), indicating that these carbohydrates are recognized as well as glucose. Thus, an assay used to screen mutant forms of s-GDH should give similar results to those obtained in FIG. 4 in that only Asp167Glu should exhibit a reduced response to xylose.

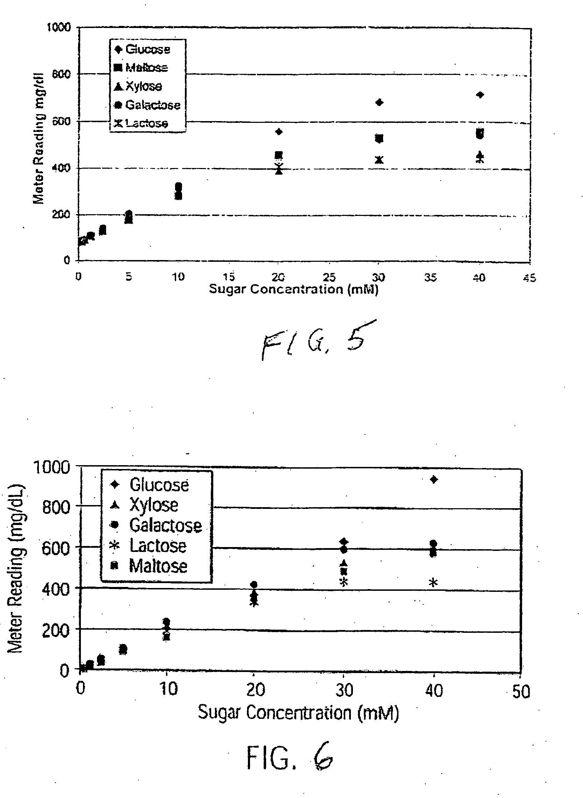

The results shown in FIG. 4 did not include testing over the whole physiologically relevant range of sugar concentration (i.e., up to 720 mg/dL or 40mM); therefore, more testing of conventional test strips coated with wild type s-GDH was done to expand the sugar concentration range. Plasma with about 80 mg/dL (or 4.4 mM) endogenous glucose was spiked with up to 40 mM each of five reducing sugars: glucose, maltose, xylose, galactose and lactose. The spiked samples were dosed onto the above test strips and glucose concentration was determined by chronoamperometry as above. The measured current response was plotted as a function of sugar concentration as shown in FIG. 5. As in FIG. 4, at less than about 10mM, wild-type s-GDH reacts equally well with each of the five reducing sugars tested. However, when tested with the DCIP/PES spectrophotometric assay described above, s-GDH has low reactivity with xylose and galactose across the same range of sugar concentrations (results not shown), indicating that the spectrophotometric assay cannot be used to model enzyme response to interfering sugars for conventional enzyme-coated test strips. As mentioned previously, the spectrophotometric assay uses enzyme-limiting conditions, whereas conventionally enzyme-coated test strips use enzyme in excess; thus, explaining the difference in sugar response with each assay format.

### Example 2 -- Test of wild-type s-GDH response to reducing sugars in a process using an electrochemical cell according to the present invention

The response of wild-type s-GDH to reducing sugars was measured using electrochemical cells (i.e., test strips) made by a web-based process in which the working electrode (gold) was slot coated with a buffered reagent solution containing 750 mM ferricyanide, 67 mM citraconate at pH 6.8, 0.1% pluronics and 75 mM sucrose. As in Example 1, an IR energy source was used to dry the reagent onto the working electrode. Electrochemical cells were formed by adhering the working electrode with dried reagent to a gold counter electrode containing dried MESA to prevent fouling of the counter electrode.

Wild-type s-GDH in 67 mM citraconate at pH 6.8 with PQQ at a 2 to 1 mole ratio of PQQ to GDH and 2 mM CaC12 was mixed with physiologically relevant concentrations (up to about 40 mM or 720 mg/dL) of one of the following sugars: glucose, xylose, galactose, maltose, or lactose. The concentration of the wild type s-GDH in each solution was adjusted such that approximately 13 activity units per electrochemical cell were used. Each enzyme/sugar-containing solution was dosed onto an electrochemical cell containing dried ferricyanide, citraconate, Pluronics and sucrose but no enzyme (see above for concentrations). The current response was measured and the average of three determinations was graphed as a function of sugar concentration, as shown in FIG. 6. The results were similar to those obtained from the s-GDH-coated strips in Example 1 (see FIG. 4) at xylose concentrations less than 10 mM in that the enzyme reacted equally well with glucose and xylose. Also, similar results to those obtained in FIG. 5 were obtained with all the reducing sugars. Thus, the assay models the response obtained with conventional wild type enzyme-coated test strips with the following advantages: (1) enzyme-coated strips do not have to be manufactured with each form of enzyme, thereby saving time and resources (equipment and technicians) and using less enzyme; and (2) buffer-based samples containing varying concentrations of reducing sugar can be used in place of biohazardous whole blood or plasma spiked with sugar.

### Example 3 - Test of Asn452Thr (a mutant form of s-GDH) response to reducing sugars in a process using an electrochemical cell according to the present invention

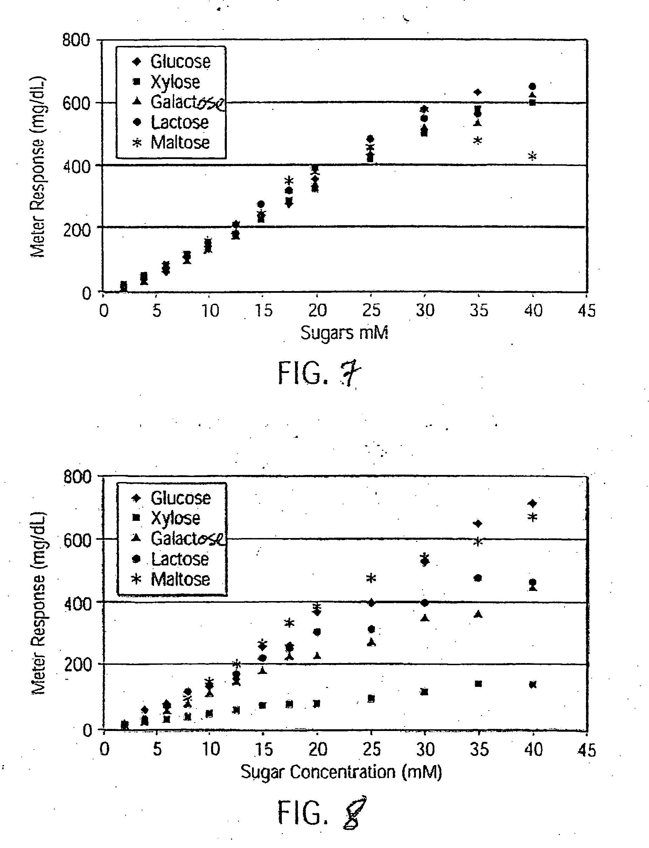

The response of Asn452Thr to reducing sugars was measured using the same lot of electrochemical cells as described in Example 2 above (i.e., with cells containing dried ferricyanide, citraconate, Pluronics and sucrose but no enzyme). Asn452Thr was mixed with physiologically relevant concentrations (up to about 40 mM or 720 mg/dL) of one of the following sugars: glucose, xylose, galactose, maltose, or lactose. The concentration of Asn452Thr in each solution was adjusted such that approximately 1-3 activity units per electrochemical cell were used. Each enzyme/sugar-containing solution was dosed onto an electrochemical cell containing dried ferricyanide, citraconate, Pluronics and sucrose but no enzyme. The current response was measured and plotted as a function of sugar concentration as shown in FIG. 7. The results were similar to those obtained with Asn452Thr-coated strips in Example 1 (see FIG. 4) at less than 10 mM xylose (i.e., at approximately three times the high therapeutic level) in that like wild type enzyme, Asn452Thr reacts equally well with glucose and xylose. The data also show that this mutant form of s-GDH reacts equally well with all the sugars tested at less than 10mM sugar. Thus, the assay mimics the results obtained with Asn452Thr-coated strips with the advantages discussed above.

### Example 4 -- Test of Asp167Glu (another mutant form of s-GDH) response to reducing sugars in a process using an electrochemical cell according to the present invention

The response of Asp167Glu to reducing sugars was measured as described in Example 3 above. Asp167Glu was mixed with physiologically relevant concentrations (up to about 40 mM or 720 mg/dL) of one of the following sugars: glucose, xylose, galactose, maltose, or lactose; and dosed onto an electrochemical cell containing dried ferricyanide, citraconate, Pluronics and sucrose but no enzyme. The current response was measured and plotted as a function of sugar concentration as shown in FIG. 8. The results were similar to those obtained from the Asp167Glu-coated strips in Example 1 (see FIG. 4) at less than 10 mM xylose (i.e., at approximately three times the high therapeutic level) in that this mutant form of s-GDH exhibits a reduced response to xylose. This mutant also reacts equally well with all the other reducing sugars tested. Thus, again; the assay beneficially mimics the results obtained with Asp167Glu-coated strips but in a quick and easy-to-use format.

The above examples demonstrate that the method can be used to model enzyme-coated strip performance and can be used to screen mutant forms of s-GDH for sugar response.

The following examples demonstrate how to use the method to measure enzyme activity.

### Example 5 -- Standard curve for an electrochemical cell dosed with wild-type s-GDH according to a process of the present invention

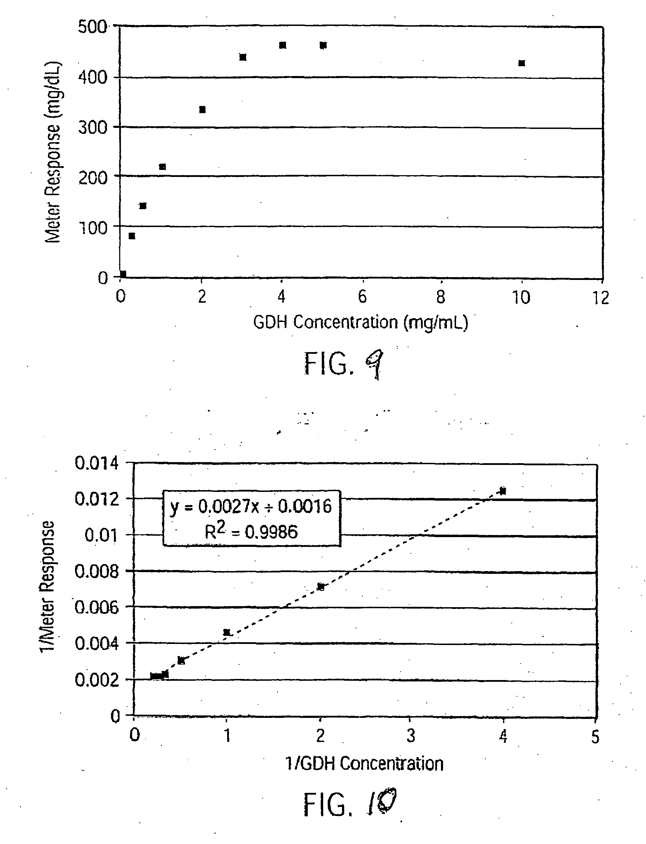

A standard curve (see FIG. 9) for wild-type s-GDH was generated by dosing electrochemical cells containing dried ferricyanide, citraconate, Pluronics and sucrose but no enzyme (see above for concentrations) with increasing concentrations of wild-type s-GDH ranging from about 0-10 mg/ml. The glucose concentration was held at 450 mg/dL. Those skilled in the art will recognize that a standard curve can also be constructed for any mutant form of s-GDH.

A double reciprocal plot (i.e., 1/response as a function of 1/enzyme concentration) was constructed from the standard curve in FIG. 9 and is shown in FIG. 10. A strong linear correlation (r2=0.9986) exists between the electrochemical cell response and the amount of wild-type s-GDH. The concentration of a test lot of enzyme can easily be determined by measuring the current response and reading the concentration off the standard curve.

Generation of a standard curve for s-GDH in an electrochemical format beneficially allows for measuring the activity of incoming enzymes used to manufacture conventional test strips, measuring the stability of enzymes coated on dry strips and troubleshooting potential strip coating problems that relate to enzyme activity.

The above results and discussion demonstrate that the present invention provides convenient and cost effective ways to characterize redox reagent system enzymes. Advantages of the subject invention include lower cost and the ability to test without the use of the blood or blood products. As such, the subject invention represents a significant contribution to the art.

## Claims

1. A method for characterizing an enzyme of a redox reagent system which comprises:
(a) applying a sample comprising a first enzyme of a redox reagent system and a known amount of an enzyme substrate to an electrochemical cell comprising an enzyme-free reagent composition comprising a mediator of said redox reagent system; and
(b) detecting an electrical signal produced by said cell.

2. The method according to claim 1, wherein said enzyme is an oxidizing enzyme.

3. The method according to claim 2, wherein said oxidizing enzyme is chosen from an oxidase and a dehydrogenase.

4. The method according to claim 1, 2, or 3, wherein said sample further comprises an enzyme cofactor.

5. The method according to claim 1, 2, 3, or 4, wherein said method further comprises using said detected electrical signal to determine analyte specificity of said first enzyme of a redox reagent system.

6. The method according to claim 5, wherein said method comprises detecting electrical signals produced from at least the following samples: (i) a sample containing a first enzyme of a redox reagent system and a known first concentration of a first enzyme substrate; (ii) a sample containing said first enzyme of a redox reagent system and a known first concentration of a second enzyme substrate; (iii) a sample containing a second enzyme of a redox reagent system and said known first concentration of said first enzyme substrate; and (iv) a sample containing said second enzyme of a redox reagent system and said known first concentration of said second enzyme substrate.

7. The method according to claim 6, wherein said method further comprises detecting electrical signals produced from the following samples:
(i) a sample containing said first enzyme of a redox reagent system and a known second concentration of said first enzyme substrate;
(ii) a sample containing said first enzyme of a redox reagent system and a known second concentration of said second enzyme substrate;
(iii) a sample containing said second enzyme of a redox reagent system and said known second concentration of said first enzyme substrate; and
(iv) a sample containing said second enzyme of a redox reagent system and said known second concentration of said second enzyme substrate.

8. The method according to claim 7, wherein said determined analyte specificity of said first enzyme of a redox reagent system is relative to said second enzyme of a redox reagent system.

9. The method according to claim 8, wherein said first enzyme of a redox reagent system is a non-naturally occurring enzyme of a redox reagent system.

10. The method according to claim 8, wherein said second enzyme of a redox reagent system is a naturally occurring enzyme of a redox reagent system.

11. The method according to claim 1, wherein said method further comprises using said detected electrical signal to determine activity of said enzyme in said sample.

12. An electrochemical cell comprising a working electrode and a reference electrode that are separated by a spacer layer to define a reaction zone; and an enzyme-free reagent composition comprising a redox reagent system mediator that is present in the reaction zone of the electrochemical cell.

13. A system comprising:
(a) an electrochemical cell comprising an enzyme-free reagent composition comprising a redox reagent system mediator; and
(b) a fluid medium comprising an enzyme of a redox reagent system and a known amount of an enzyme substrate.

## Patentansprüche

1. Verfahren zur Charakterisierung eines Enzyms eines Redox-Reagenz-Systems, welches umfasst:
(a) Aufbringen einer Probe, die ein erstes Enzym eines Redox-Reagenz-Systems und eine bekannte Menge eines Enzymsubstrats umfasst, auf eine elektrochemische Zelle, die eine enzymfreie Reagenz-Zusammensetzung umfasst, die einen Mediator für das Redox-Reagenz-System umfasst; und
(b) Nachweisen eines von der Zelle erzeugten elektrischen Signals.

2. Verfahren nach Anspruch 1, wobei das Enzym ein oxidierendes Enzym ist.

3. Verfahren nach Anspruch 2, wobei das oxidierende Enzym ausgewählt ist aus einer Oxidase und einer Dehydrogenase.

4. Verfahren nach Anspruch 1, 2 oder 3, wobei die Probe weiter einen Enzym-Cofaktor umfasst.

5. Verfahren nach Anspruch 1, 2, 3 oder 4, wobei das Verfahren weiter die Verwendung des nachgewiesenen elektrischen Signals umfasst, um die Analytspezifität des ersten Enzyms eines Redox-Reagenz-Systems zu bestimmen.

6. Verfahren nach Anspruch 5, wobei das Verfahren das Nachweisen elektrischer Signale umfasst, die aus wenigstens den folgenden Proben erzeugt sind: (i) einer Probe, die ein erstes Enzym eines Redox-Reagenz-Systems und eine bekannte erste Konzentration eines ersten Enzymsubstrats enthält; (ii) einer Probe, die das erste Enzym eines Redox-Reagenz-Systems und eine bekannte erste Konzentration eines zweiten Enzymsubstrats enthält; (iii) einer Probe, die ein zweites Enzym eines Redox-Reagenz-Systems und die bekannte erste Konzentration des ersten Enzymsubstrats enthält; und (iv) einer Probe, die das zweite Enzym eines Redox-Reagenz-Systems und die bekannte erste Konzentration des zweiten Enzymsubstrats enthält.

7. Verfahren nach Anspruch 6, wobei das Verfahren weiter das Nachweisen elektrischer Signale umfasst, die aus den folgenden Proben erzeugt sind:
(i) einer Probe, die das erste Enzym eines Readox-Reagenz-Systems und eine bekannte zweite Konzentration des ersten Enzymsubstrats enthält;
(ii) einer Probe, die das erste Enzym eines Redox-Reagenz-Systems und eine bekannte zweite Konzentration des zweiten Enzymsubstrats enthält;
(iii) einer Probe, die das zweite Enzym eines Redox-Reagenz-Systems und die bekannte zweite Konzentration des ersten Enzymsubstrats enthält; und
(iv) einer Probe, die das zweite Enzym eines Redox-Reagenz-Systems und die bekannte zweite Konzentration des zweiten Enzymsubstrats enthält.

8. Verfahren nach Anspruch 7, wobei die bestimmte Analytspezifität des ersten Enzyms eines Redox-Reagenz-Systems relativ ist zum zweiten Enzym eines Redox-Reagenz-Systems.

9. Verfahren nach Anspruch 8, wobei das erste Enzym eines Redox-Reagenz-Systems ein nicht natürlich vorkommendes Enzym eines Redox-Reagenz-Systems ist.

10. Verfahren nach Anspruch 8, wobei das zweite Enzym eines Redox-Reagenz-Systems ein natürlich vorkommendes Enzym eines Redox-Reagenz-Systems ist.

11. Verfahren nach Anspruch 1, wobei das Verfahren weiter die Verwendung des nachgewiesenen elektrischen Signals umfasst, um die Aktivität des Enzyms in der Probe zu bestimmen.

12. Elektrochemische Zelle, die ein Arbeitselektrode und eine Referenzelektrode, die durch eine Abstandsschicht getrennt sind, um eine Reaktionszone zu definieren; und eine enzymfreie Reagenz-Zusammensetzung umfasst, die einen Redox-Reagenz-System-Mediator umfasst, der in der Reaktionszone der elektrochemischen Zelle vorliegt.

13. System, umfassend:
(a) eine elektrochemische Zelle, die eine enzymfreie Reagenz-Zusammensetzung umfasst, die einen Redox-Reagenz-System-Mediator umfasst; und
(b) ein fließfähiges Medium, das ein Enzym eines Redox-Reagenz-Systems und eine bekannte Menge eines Enzymsubstrats umfasst.

## Revendications

1. Méthode de caractérisation d'une enzyme d'un système réactif redox qui comprend :
(a) l'application d'un échantillon comprenant une première enzyme d'un système réactif redox et une quantité connue d'un substrat enzymatique à une cellule électrochimique comprenant une composition de réactif dépourvue d'enzyme comprenant un médiateur dudit système réactif redox ; et
(b) la détection d'un signal électrique produit par ladite cellule.

2. Méthode selon la revendication 1, dans laquelle ladite enzyme est une enzyme oxydante.

3. Méthode selon la revendication 2, dans laquelle ladite enzyme oxydante est choisie parmi une oxydase et une déshydrogénase.

4. Méthode selon la revendication 1, 2 ou 3 dans laquelle ledit échantillon comprend en outre un cofacteur enzymatique.

5. Méthode selon la revendication 1, 2, 3 ou 4, dans laquelle ladite méthode comprend en outre l'utilisation dudit signal électrique détecté pour déterminer la spécificité de l'analyte de ladite première enzyme d'un système réactif redox.

6. Méthode selon la revendication 5, ladite méthode comprenant la détection de signaux électriques produits à partir d'au moins les échantillons suivants :
(i) un échantillon contenant une première enzyme d'un système réactif redox et une première concentration connue d'un premier substrat enzymatique ;
(ii) un échantillon contenant ladite première enzyme d'un système réactif redox et une première concentration connue d'un deuxième substrat enzymatique ;
(iii) un échantillon contenant une deuxième enzyme d'un système réactif redox et ladite première concentration connue dudit premier substrat enzymatique ; et
(iv) un échantillon contenant ladite deuxième enzyme d'un système réactif redox et ladite première concentration connue dudit deuxième substrat enzymatique.

7. Méthode selon la revendication 6, ladite méthode comprenant en outre la détection de signaux électriques produits à partir des échantillons suivants :
(i) un échantillon contenant ladite première enzyme d'un système réactif redox et une deuxième concentration connue dudit premier substrat enzymatique ;
(ii) un échantillon contenant ladite première enzyme d'un système réactif redox et une deuxième concentration connue dudit deuxième substrat enzymatique :
(iii) un échantillon contenant ladite deuxième enzyme d'un système réactif redox et ladite deuxième concentration connue dudit premier substrat enzymatique ; et
(iv) un échantillon contenant ladite deuxième enzyme d'un système réactif redox et ladite deuxième concentration connue dudit deuxième substrat enzymatique.

8. Méthode selon la revendication 7, dans laquelle ladite spécificité d'un analyte déterminé de ladite première enzyme d'un système réactif redox est relative à ladite deuxième enzyme d'un système réactif redox.

9. Méthode selon la revendication 8, dans laquelle ladite première enzyme d'un système réactif redox est une enzyme non présente à l'état naturel d'un système réactif redox.

10. Méthode selon la revendication 8, dans laquelle ladite deuxième enzyme d'un système réactif redox est une enzyme présente à l'état naturel d'un système réactif redox.

11. Méthode selon la revendication 1, ladite méthode comprenant en outre l'utilisation dudit signal électrique détecté pour déterminer l'activité de ladite enzyme dans ledit échantillon.

12. Cellule électrochimique comprenant une électrode de travail et une électrode de référence qui sont séparées par une couche d'espacement pour définir une zone réactionnelle ; et une composition de réactif sans enzyme comprenant un médiateur du système réactif redox qui est présent dans la zone réactionnelle de la cellule électrochimique.

13. Système comprenant :
(a) une cellule électrochimique comprenant une composition de réactif sans enzyme comprenant un médiateur du système réactif redox ; et
(b) un milieu fluide comprenant une enzyme d'un système réactif redox et une quantité connue d'un substrat enzymatique.
